# FASCICULE DE BREVET EUROPEEN

(11) **EP 0 606 026 B1**
(45) Date de publication et mention de la délivrance du brevet: **26.03.1997**
(21) Numéro de dépôt: 93402901.8
(22) Date de dépôt: 30.11.1993
(51) Int. Cl.: G01J 1/42

(54) **Procédé et dispositif de contrôle d'appareils d'émission de radiations électromagnétiques**
Verfahren und Vorrichtung zum Überwachen von elektromagnetische Strahlung emittierenden Gegenständen
Controlling method and device for electromagnetic radiation emitting apparatuses

(30) Priorité: 22.12.1992 FR 9215486
(43) Date de publication de la demande: 13.07.1994
(73) Titulaire: Decupper, Jean, F-06400 Cannes (FR)
(72) Inventeur: Decupper, Jean, F-06400 Cannes (FR)
(74) Mandataire: Wagret, Frédéric

(56) Documents cités:
- EP-A- 0 392 442
- FR-A- 2 639 433
- US-A- 3 427 489
- US-A- 3 629 587
- US-A- 4 061 922
- US-A- 4 103 167
- US-A- 4 659 930
- US-A- 4 885 471
- PATENT ABSTRACTS OF JAPAN vol. 11, no. 243 (P-603)(2690) 8 Août 1987& JP-A-62 054 129 ( SEIKO EPSON ).

## Description

La présente invention concerne un procédé et un dispositif en vue de la détection et du contrôle de l'intensité d'une émission électromagnétique de très courte longueur d'onde, se situant au voisinage de la lumière visible, telle que des émissions d'onde dans l'ultraviolet à des fins germicides d'assainissement d'ambiance.

Bien que l'invention soit ci-après décrite en rapport avec une application plus spécifique et plus particulière, à savoir le contrôle de tubes d'émission de radiations ultraviolettes germicides, elle est applicable de raçon plus générale au contrôle d'appareils d'émission de radiations électromagnétiques se situant notamment au voisinage de la lumière visible.

L'application plus spécifique qui sera ici prise en considération concerne un contrôle afin d'alerter immédiatement le personnel de service lorsqu'un appareil germicide, émettant des radiations ultraviolettes, tombe en dessous d'un seuil minimum d'efficacité.

On sait que de tels appareils constitués d'un tube électrique fluorescent émettant des radiations dans l'ultraviolet d'une longueur d'onde très précise sont utilisés notamment dans les milieux sensibles et à risque tels que des locaux médicaux ou hospitaliers, chambres de conservation, travail ou conditionnement de produits alimentaires, etc.

Les radiations ultraviolettes de la longueur d'onde connues ( 2 537 Angströms) sont utilisées pour leurs propriétés spécifiquement germicides et bactériostatiques.

Et on utilise à cet effet des boîtiers contenant les tubes émetteurs de radiations (en évitant que ces radiations puissent atteindre la vue du personnel), l'air étant susceptible notamment d'être mis en circulation naturelle ou forcée autour des tubes des façon à provoquer au fur et à mesure de cette circulation la destruction des germes présents sur les micro-poussières en suspension dans l'air.

On sait également que de tels tubes générateurs de radiations U.V. sont soumis à un vieillissement au terme duquel leur intensité et leur débit de radiations chutent, tout comme par suite de dépôts; de salissures ou poussières, ils peuvent perdre une grande partie de leur efficacité.

Comme ces tubes émettent, parallèlement aux radiations ultraviolettes, des rayonnements dans la lumière visible, notamment bleue, il n'est pas possible de détecter a priori et par un simple coupe d'oeil l'état de fonctionnement ou le degré d'efficacité du rayonnement, puisque seule la lumière bleue visible à l'oeil peut être détectée et cette lumière, en soi totalement inefficace et sans propriétés germicides, n'est nullement un indice de l'existence et de l'importance des émissions ultraviolettes parallèles.

De sorte que fréquemment, le tube U.V. reste en place, en continuant à émettre sa lumière bleutée, alors qu'il est devenu totalement inefficace sans aucune action germicide.

On est conduit dans la technique actuelle pour déceler l'état de non fonctionnement d'un tube U.V., par suite de son vieillissement ou de dépôt de salissures formant écran, à soumettre ces tubes à une maintenance périodique sous forme d'un examen individuel permettant de vérifier à l'occasion de cette opération systématique les appareils devant être remplacés, rénovés ou entretenus.

L'efficacité de la maintenance de ces appareils dépend par conséquent de la vigilance du personnel et tout manquement à cette discipline entraîne des conséquences qui peuvent être fatales pour les malades, ne serait-ce que parce qu'entre deux contrôles ponctuels, un ou plusieurs tubes peuvent devenir totalement inefficaces, inefficacité qui ne sera détectée éventuellement qu'à l'occasion du contrôle ultérieur, laissant par conséquent subsister une période de temps pendant laquelle la protection de l'air ne sera pas assurée.

En outre, la vérification est effectuée en utilisant un dosimètre portatif qui nécessite l'ouverture du carter de l'appareil, les tubes U.V. étant alors nécessairement en action; on expose pendant un temps déterminé le dosimètre au champ des radiations émises par le tube, période pendant laquelle le personnel de service reste donc exposé aux radiations U.V.; ces conditions de travail ne sont pas satisfaisantes car elles sont dangereuses pour la vue et pour le visage de l'opérateur; et pour ces raisons, ces vérifications périodiques sont peu incitatrices pour le personnel hospitalier.

On voit qu'il existe donc un besoin pour une méthodologie et un appareil permettant d'éviter d'une part de laisser subsister un appareil en fonctionnement apparent, alors qu'il est en fait inactif, et d'autre part d'exposer à l'occasion des vérifications le personnel de service aux radiations U.V..

Le brevet EP 392 442 concerne un système de mesure de degré d'irradiation au soleil d'un individu, destiné principalement à avertir celui-ci si le seuil maximum de bronzage qu'il est susceptible de supporter, est dépassé. Le système comprend deux photodiodes sensibles au moins aux U.V. et un filtre faisant écran aux radiations U.V. disposé devant l'une des deux photodiodes. La différence des courants issus des photodiodes, est calculée et l'intensité des radiations U.V. est affichée. L'intensité cumulée est comparée au seuil maximum.

Ce dispositif connu, nécessite l'utilisation de cellules sensibles aux U.V..

Le brevet US 3 629 587 décrit un dispositif d'évaluation d'une action germicide, utilisant une cellule photoélectrique sensible aux U.V. en association avec un filtre passe-bande U.V.. Ce dispositif connu ne supprime pas les radiations voisines des radiations U.V., obtenue par la différence des courants.

On connaît par le brevet US 4 103 167 un système de purification de liquide par radiation U.V., similaire à celui décrit par US 3 629 587, mais sans filtre.

Le brevet US 3 427 489 se rapporte à un dispositif comportant une photodiode sensible aux rayonnements visibles de 4.000 à 7.000 Angström issus de la transformation par une couche de phosphore de radiations U.V. de 2.537 Angström.

L'invention vise précisément à répondre aux objets mentionnés précédemment et elle remédie aux inconvénients et aux déficiences de la technique antérieure telle qu'elle a été ci-dessus évoquée.

A cet effet, l'invention concerne un procédé pour le contrôle du débit et de l'intensité d'une émission électromagnétique notamment de très courte longueur d'onde, au voisinage de la lumière visible, telle qu'une émission d'ondes dans l'ultraviolet, caractérisé en ce que l'on expose aux radiations à contrôler un premier et un second capteur constitués chacun d'un transducteur notamment du type photovoltaïque, tandis que l'on interpose devant lesdits capteurs une série de filtres un premier filtre ou groupe de filtres étant aptes à atténuer ou arrêter les radiations marginales voisines de la longueur d'onde surveillée et se situant dans le spectre de la lumière visible, un second filtre étant apte à transformer l'émission du rayonnement à surveiller en lumière visible apte à être détecté par la première cellule photoélectrique, tandis que la seconde cellule photoélectrique comporte seule un filtre occultant le rayonnement visible résultant de ladite transformation, le rayonnement visible résultant de cette transformation n'étant reçu que par la première cellule, on compare les courants débités par chacun de ces transducteurs, le différentiel entre les deux courants étant proportionnel à l'intensité du rayonnement surveillé, ce qui permet de contrôler ou d'afficher cette intensité, on détermine un seuil minimum de consigne dudit différentiel, en dessous duquel est actionné un moyen déclenchant une intervention automatique telle que la coupure d'alimentation de l'émetteur soumis à surveillance.

Selon un développement du procédé ci-dessus, on contrôle en permanence les émissions de telle sorte que toute défaillance due à un vieillissement ou pour toute autre raison, peut être immédiatement détectée et signalée lorsqu'elle atteint un niveau prédéterminé.

L'invention concerne également un dispositif de détection , contrôle et surveillance, d'un émetteur de radiations électromagnétiques, et du type comprenant deux capteurs, une série de filtres sensibles aux radiations à contrôler, et des moyens de mesure et comparaison entre les courants débités par les capteurs, caractérisé en ce que ce dispositif est agencé pour la mise en oeuvre du procédé.

L'invention porte sur un dispositif permettant de détecter, contrôler et surveiller le dispositif permettant de détecter, contrôler et surveiller en permanence un émetteur de radiations électromagnétiques de longueur d'onde se situant au voisinage de la lumière visible, notamment dans l'ultraviolet et utilisées plus spécialement à des fins germicides et d'assainissement d'ambiance et d'atmosphère, plus particulièrement en milieux sensibles tels que locaux médicaux, hospitaliers ou de traitement de produits alimentaires, installation de climatisation d'air ou piscines, et le dispositif est caractérisé en ce qu'il comporte deux cellules photoélectriques disposées dans le champ du rayonnement surveillé, une pluralité de filtres étant interposés dans ledit champ entre l'émetteur et les cellules , et un premier filtre ou groupe de filtres étant aptes à atténuer ou arrêter les radiations marginales voisines de la longueur d'onde surveillée et se situant dans le spectre de la lumière visible, un second filtre étant apte à transformer l'émission du rayonnement à surveiller en lumière visible apte à être détecté par la première cellule photoélectrique, tandis que la seconde cellule photoélectrique comporte seule un filtre occultant le rayonnement visible résultant de ladite transformation, le rayonnement visible résultant de cette transformation n'étant reçu que par la première cellule, les courants voltaïques débités par les deux cellules photoélectriques étant d'intensité différente en raison de cette différence, ceci pour le fonctionnement normal de l'émetteur, et les deux courants voltaïques étant recueillis aux bornes d'un comparateur programmé pour basculer un relais lorsque le différentiel entre les deux courants descend en dessous d'une valeur de consigne déterminée.

Le dispositif comporte un relais de contrôle et de surveillance, apte à couper l'alimentation de l'émetteur et à actionner parallèlement un ensemble de signalisations.

De préférence, le dispositif de contrôle et de surveillance est associé en permanence à un émetteur ultraviolet constitué lui-même d'un tube d'émission ultraviolette intégré dans un carter de protection.

Selon une variante, le dispositif est monté à demeure à proximité immédiate de l'émetteur de radiations à surveiller et au moins les cellules photoélectriques comportent des moyens propres à assurer leur déplacement, depuis une position effacée vers une position active dans laquelle elles sont situées dans le champ du rayonnement à surveiller.

Selon une forme de réalisation plus particulière, l'appareil comporte un ensemble de composants monté sur un socle lui-même aimanté permettant de le positionner sur le carter métallique du boîtier d'un appareil à émission de radiations U.V..

Avantageusement, l'appareil de contrôle comporte un potentiomètre à axe fendu pour le réglage du seuil de sensibilité, c'est-à-dire de l'intensité de radiations en dessous duquel l'intensité des radiations est considérée comme insuffisance et inefficace.

L'appareil de contrôle comporte avantageusement un relais apte à couper sa propre alimentation lorsque l'émission soumise à surveillance descend en dessous du seuil de consigne déterminé, l'appareil étant monté en série en amont de l'appareil émetteur, la coupure de l'alimentation entraînant par conséquent l'arrêt de l'appareil émetteur lui-même.

Selon une variante, l'appareil comporte un ensemble porteur des composants électroniques, de la connectique, des moyens d'alarme et de moyens de protection rassemblés sur un socle comportant une semelle aimantée permettant le positionnement sur le carter métallique de l'appareil d'émission U.V., et le socle est associé à un boîtier séparé relié audit socle par une liaison filaire, le boîtier comportant les deux cellules et les filtres, aptes à être positionnés dans le champ des radiations U.V., ce boîtier comportant également une semelle aimantée pour sa fixation.

Selon enfin une variante de réalisation, chaque appareil de contrôle est relié par un circuit de signalisation à une console centrale de commande et de surveillance regroupant les signaux provenant de l'ensemble des appareils U.V. appartenant à une même unité.

D'autres caractéristiques et avantages de l'invention ressortiront de la description qui va suivre et qui est donnée en rapport avec une forme particulière de réalisation présentée à titre d'exemple non limitatif et en se référant aux dessins annexés, dans lesquels:
- la figure 1 représente une vue schématique et de principe de l'ensemble des moyens mis en oeuvre dans le cadre de la présente invention, tandis que
- la figure 2 représente une vue schématique en perspective d'un exemple de réalisation de l'appareil de contrôle conforme à l'invention;
- la figure 3 représente un système de surveillance d'une batterie de tubes.

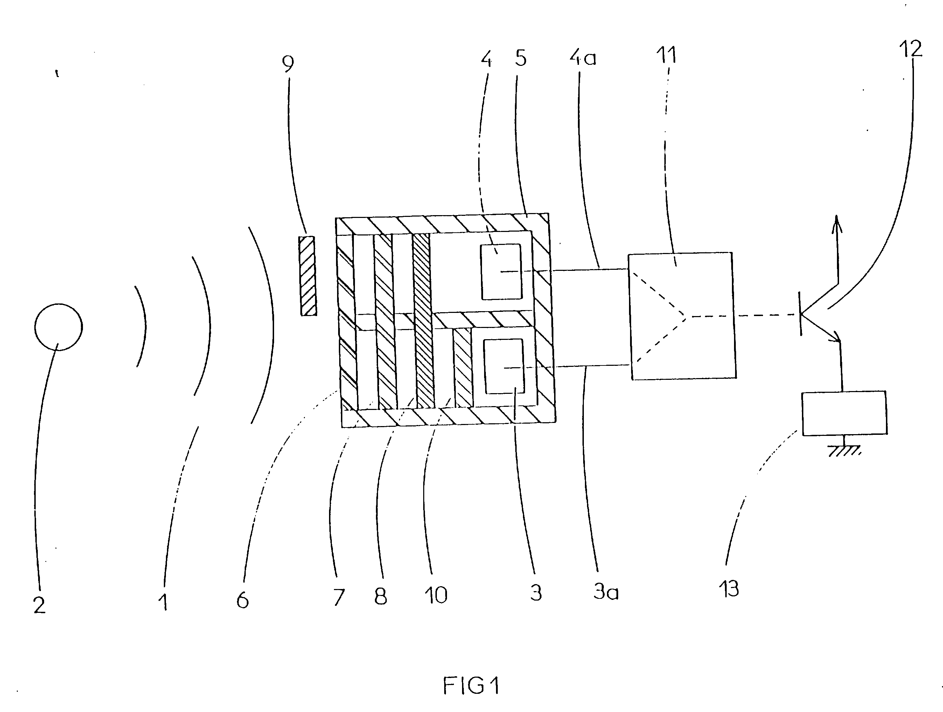

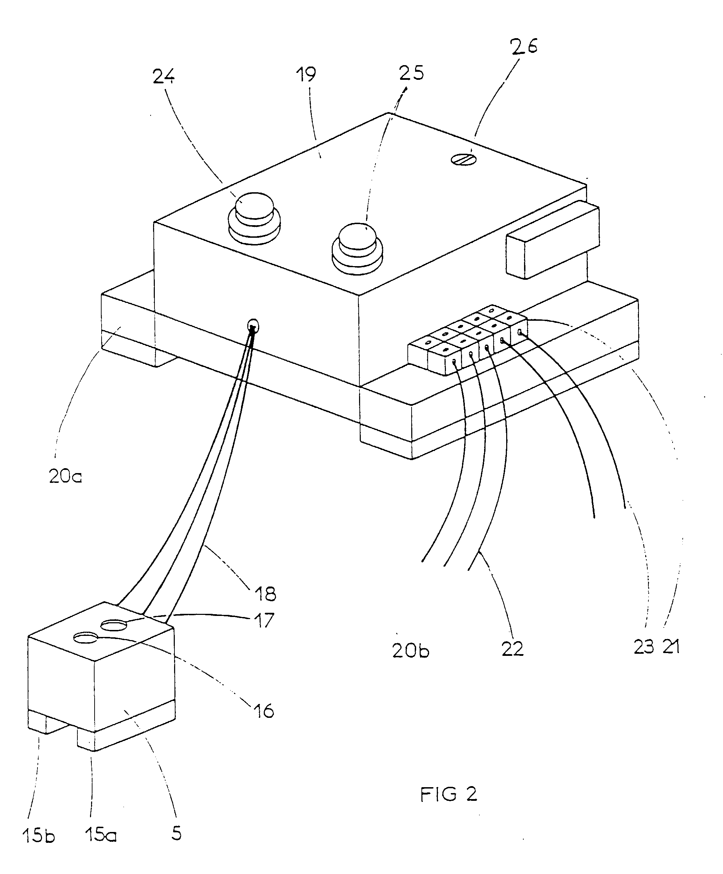

En se référant à l'ensemble des figures 1 et 2, on voit qu'on a représenté schématiquement un émetteur de radiations sous forme d'un tube U.V. de type connu.

Ce tube U.V. est lui-même inséré dans un carter et il est mis en place de façon systématique dans des locaux à ambiance sensible tels que des locaux médicaux, hospitaliers, locaux collectifs, locaux sanitaires, cuisines, ateliers de préparation de conditionnement de produits alimentaires ou analogues.

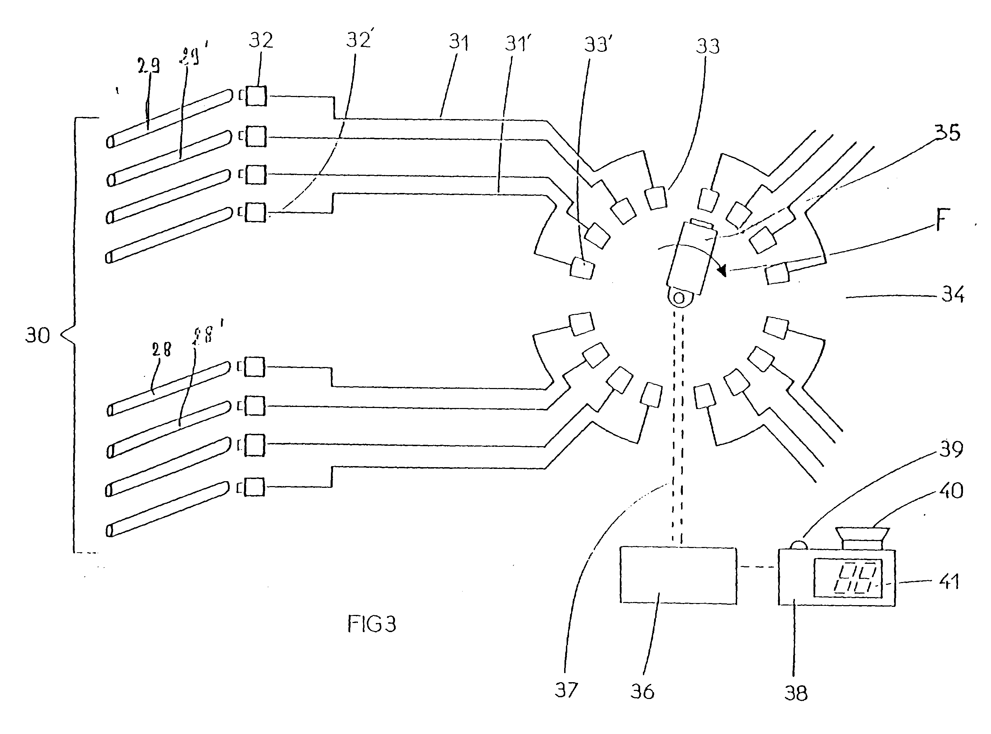

On comprend que la principale difficulté dans le cadre du problème ici évoqué consiste à éliminer l'influence des lumières de longueur d'onde voisine dans le spectre du visible et notamment de la lumière bleutée qui est émise en permanence par le tube, ceci indépendamment de son efficacité quant à l'émission de la radiation ultraviolette effective pour son action bactéricide; la figure 3 illustre une variante du dispositif selon l'invention adaptée à la surveillance simultanée et conjuguée d'une pluralité de tubes émetteurs de radiations et disposée en batterie.

Dans le cadre de l'invention, on expose aux radiations du tube 2 deux cellules photoélectriques physiquement et électroniquement identiques à savoir la première cellule photoélectrique 3 et la seconde cellule photoélectrique 4 jumelées entre elles et susceptibles d'être montées dans un boîtier commun 5.

Ces cellules sont exposées de façon à recevoir les radiations 1 provenant du tube 2.

Mais devant les cellules, sont interposés une pluralité de premiers filtres 6, 7, 8 destinés à arrêter, pour en éliminer l'effet parasite, les radiations se situant dans la lumière visible, tandis qu'une de ces plaques filtrantes est prévue pour transformer les radiations ultraviolettes en lumière visible, notamment en lumière verte.

En outre, la plaque filtrante 9 interposée devant la cellule ou seconde cellule photoélectrique 4 arrête les radiations se situant dans la longueur d'onde surveillée, c'est-à-dire les radiations ultraviolettes.

De sorte que la première cellule 3 est rendue sensible aux radiations ultraviolettes, c'est-à-dire à la lumière verte visible résultant de la transformation des radiations ultraviolettes en lumière verte.

La seconde cellule 4 est elle-même insensible aux radiations ultraviolettes, lesquelles ne peuvent l'atteindre ayant été filtrées au niveau de la paroi filtrante 9.

Les deux cellules photoélectriques 3 et 4 sont en outre insensibilisées ou rendues peu sensibles à la lumière visible, laquelle est éliminée par les filtres interposés en amont.

On peut en outre ajouter à cet ensemble de filtration la plaque filtrante 10 située en amont de la première cellule 3, cette quatrième source de filtration étant rendue transparente à la seule lumière verte résultant de la transformation des radiations ultraviolettes.

Les deux cellules 3 et 4 débitent un courant voltaïque qui résulte de la transformation des énergies lumineuses reçues en énergie électrique.

Ces deux courants sont transmis à un circuit intégré 11, lequel joue le rôle de comparateur.

On comprend que les deux cellules jumelées 3 et 4 débitent un courant nécessairement différent, tout au moins pendant la période d'émission normale du tube 2 puisqu'une cellule reçoit, sous forme de lumière visible verte, l'énergie provenant des radiations ultraviolettes, tandis que cette énergie est éliminée au niveau de la seconde cellule 4.

Et la différence ou le différentiel entre les deux courants provenant des cellules 3 et 4, et transmis par les circuits 3a et 4a, peut être détecté et analysé au niveau du comparateur 11.

Plus le différentiel sera élevé, et plus le rayonnement électromagnétique surveillé pourra être considéré comme satisfaisant.

A la limite, lorsque le rayonnement électromagnétique surveillé, par exemple le rayonnement ultraviolet disparaît, la première cellule photoélectrique 3 ne reçoit pas plus d'énergie que la cellule témoin 4; et on peut considérer que cette absence de différentiel traduit par conséquent l'inexistence de radiations ultraviolettes émises par le tube.

Il est ainsi commode d'afficher une valeur de consigne à ce différentiel, valeur de consigne en dessous de laquelle on retient l'émission de radiations U.V. comme insatisfaisante et inacceptable.

Le transistor 12 est alors basculé pour actionner un relais 13.

Ce relais peut être de tout type convenable et peut correspondre à la mise en activité d'un appareil de signalisation visuel ou sonore; ce peut être un bruiteur 14 tel que représenté sur la figure 2.

Le relais 13 peut transmettre tout signal d'alarme, notamment à une console centrale depuis une salle de surveillance.

Selon une variante, le relais 13 est inséré sur l'alimentation de l'appareil lui-même; l'appareil de contrôle étant monté en série et en amont de l'appareil d'émission de radiations, notamment de l'alimentation du tube U.V.; de sorte que l'appareil s'auto-désalimente lui-même en interrompant par conséquent l'alimentation du tube U.V.

On notera déjà que dans ce cas, une signalisation se trouve ainsi automatiquement mise en action puisque l'extinction du tube U.V. faisant disparaître la lumière visible bleutée signale au personnel de service la mise en position inactive du tube par suite de sa défaillance en tant qu'émetteur de radiations U.V.

On voit sur la figure 2 représenté en perspective un ensemble conforme à l'invention et comportant d'une part un boîtier 5 monté sur une semelle aimantée ou double semelle aimantée 15a, 15b permettant de fixer simplement sans perçage le boîtier à l'intérieur du carter d'un appareil émetteur de radiations U.V., les ouvertures 16 et 17 étant exposées face au tube U.V. pour permettre la réception des radiations par les cellules photoélectriques 3 et 4 disposées à l'intérieur du boîtier 5.

La liaison filaire 18 correspondant aux circuits 3a et 4a aboutit au socle 19 monté lui-même sur les semelles aimantées 20a et 20b permettant sa fixation à l'intérieur ou à l'extérieur du carter de l'appareil émetteur de U.V.

Le bornier 21 permet le raccordement pour la réception de l'alimentation depuis le secteur 22 et le départ de l'alimentation 23 vers les tubes U.V.

Le contacteur manuel 24 sous forme de bouton-poussoir permet d'arrêter le bruiteur et éventuellement de réarmer l'appareil après mise en alarme; un fusible de protection 25 est également positionné sur le boîtier.

Tandis que le potentiomètre situé à l'intérieur du socle 19 peut être actionné et réglé par sa sortie à axe fendu de type connu 26.

L'invention permet ainsi de réaliser avec des moyens économiques un ensemble de surveillance permanent de tubes U.V. mis en place dans un milieu sensible et soumis à l'action de rayonnements germicides.

Cette surveillance permet d'éviter les soucis, les inconvénients sur le plan social et médical des rondes de maintenance, puisque l'appareil se désactive automatiquement dès qu'il a cessé d'être efficace en se signalant lui-même à l'attention du personnel; de sorte que normalement, un tube ayant cessé d'être efficace sera rapidement repéré et remplacé; sans que le personnel soit exposé aux radiations ou ait à faire une intervention de routine représentant une perte de temps dans la mesure où la plupart des appareils sont en bon état.

On réalise ainsi dans un équipement hospitalier ou agro-alimentaire une amélioration des conditions de travail, une plus grande efficacité dans la surveillance bactériostatique du milieu et une économie de main-d'oeuvre.

Cette application pourra notamment être mise en oeuvre dans le cas de batterie de tubes réunis et destinée à assurer l'assainissement et la stérilisation d'un fluide traversant la zone d'émission.

L'invention sera également applicable à des tubes émetteurs de radiations ultraviolettes disposés pour agir sur un flot ou un circuit d'eau inséré dans un ensemble de filtration et d'assainissement d'eau de piscine.

On comprend que dans tous ces cas il n'est pas possible de décentraliser la surveillance au niveau de chaque tube, ces derniers étant disposés dans une zone en principe difficilement accessible.

Il est donc nécessaire dans ce cas de déporter la signalisation et éventuellement la détection par rapport à l'appareil surveillé.

Il est donc alors nécessaire de disposer à proximité du tube une sonde réceptrice des radiations et de transporter ces dernières par un réseau approprié jusqu'à un poste central d'où les ondes ou données peuvent être traitées en vue de repérer les déflexions et de déclencher une alarme.

Selon la figure 3, on a représenté une variante du dispositif pour la télécommande d'une pluralité de tubes émetteurs de radiations d'une longueur déterminée et dans lesquels chaque tube émetteur est relié à un poste central de surveillance par un guide d'onde, notamment un câble de fibre optique connu en soi et spécifique à chaque tube, une extrémité distale du guide d'onde étant apte à recevoir la radiation depuis un tube déterminé, tandis qu'une extrémité proximale du guide d'onde étant apte à délivrer cette radiation au niveau d'un poste central de surveillance, ce dernier étant ainsi apte à recevoir une partie des radiations émises par chaque tube et transmises par ledit guide d'onde, le poste central comporte en outre une double cellule photoélectrique conforme aux caractéristiques ci-dessus et il est pourvu en outre de moyens mécaniques propres à positionner la double cellule en position d'interrogation successivement devant chacune des extrémités proximales du guide d'onde.

On voit ainsi sur la figure 3 que les tubes 29, 29', 28, 28' sont regroupés pour constituer ici une batterie 30.

Chaque tube est associé à un guide d'onde 31, 31' comportant une extrémité distale 32, 32' disposée face à chacun des tubes que ce guide dessert.

L'extrémité opposée 33, 33' de ce guide d'onde étant située au niveau du poste central 34.

Dans ces conditions, chaque guide d'onde 31 capte une émission au niveau de son extrémité distale ou d'entrée 32, située à proximité d'un tube particulier et cette radiation est réémise au niveau de l'extrémité proximale 33' au sein du poste central 34.

Les extrémités distales de tous les guides d'onde sont disposées par exemple ici en cercle.

Un ensemble de cellules jumelées 35 est monté sur un bras rotatif disposé radialement au centre de la couronne formée par l'ensemble des extrémités proximales des guides d'onde.

Cet ensemble de cellules jumelées 35 est associé à des moyens mécaniques, par exemple un moteur électrique qui permet d'entraîner sa rotation angulaire selon un mouvement pas-à-pas représenté sur la figure 3 par la flèche F.

On voit dans ces conditions que l'ensemble de cellules 35 peut être mis en position d'interrogation successivement face à chacune des extrémités proximales d'un des guides d'onde correspondant à un tube.

A l'occasion de chacune de ces interrogations au contrôle la cellule photoélectrique est apte à saisir les radiations et à les transformer pour donner des courants voltaïques de comparaison selon ce qui a été décrit précédemment.

Ces courants voltaïques sont transmis à un comparateur 36 par les circuits 37 et déclenchent comme précédemment décrit dans le cas où la distorsion entre les deux signaux tombe en dessous d'un certain seuil, à une alarme.

Le signale d'alarme est transmis au bloc 38, lequel est pourvu d'une signalisation lumineuse 39, éventuellement d'un bruiteur 40 ainsi que d'un ensemble d'affichage 41 permettant de signaler par un repère celui des appareils dont l'insuffisance a été détectée.

On comprend que cette variante permettant de surveiller des tubes en batterie peut être remplacée par un système dans lequel au niveau de chaque tube serait positionnée une cellule double; et les courants voltaïques recueillis aux bornes de sortie des cellules jumelées seraient envoyés au poste central pour y être traités, le poste central étant apte successivement à comparer les courants de sortie de chaque ensemble de cellules jumelées pour, en cas de différence tombant en dessous d'un seuil déterminé, déclencher également une alarme.

L'invention permet donc par ce développement de surveiller de façon décentralisée et déportée les tubes qui se trouveraient regroupés dans un ensemble technique difficilement accessible, rendant par conséquent délicates les opérations de surveillance locale.

Ainsi, l'accès à la zone peut être limité au cas très précis où l'alarme ayant détecté l'insuffisance d'action du tube, ce dernier doit être remplacé.

Avantageusement ledit ensemble (35) de transducteurs photovoltaïques est relié à un comparateur apte à comparer les tensions recueillies aux bornes de sortie desdits transducteurs photovoltaïques et à déclencher un signal caractérisant l'insuffisance d'efficacité du tube dans le guide d'onde (31) et situé, par son extrémité proximale (33) en position d'interrogation devant l'ensemble (35) de transducteurs photovoltaïques.

## Revendications

1. Procédé pour le contrôle du débit et de l'intensité d'une émission électromagnétique notamment de très courte longueur d'onde, au voisinage de la lumière visible, telle qu'une émission d'ondes dans l'ultraviolet,
caractérisé en ce que l'on expose aux radiations à contrôler un premier (3) et un second (4) capteur constitués chacun d'un transducteur notamment du type photovoltaïque, tandis que l'on interpose devant lesdits capteurs une série de filtres (6, 10), un premier filtre ou groupe de filtres (7, 8) étant aptes à atténuer ou arrêter les radiations marginales voisines de la longueur d'onde surveillée et se situant dans le spectre de la lumière visible, un second filtre (10) étant apte à transformer l'émission du rayonnement à surveiller en lumière visible apte à être détecté par la première cellule photoélectrique (3), tandis que la seconde cellule photoélectrique (4) comporte seule un filtre (9) occultant le rayonnement visible résultant de ladite transformation, le rayonnement visible résultant de cette transformation n'étant reçu que par la première cellule, on compare les courants débités par chacun de ces transducteurs, le différentiel entre les deux courants étant proportionnel à l'intensité du rayonnement surveillé, ce qui permet de contrôler ou d'afficher cette intensité, on détermine un seuil minimum de consigne dudit différentiel, en dessous duquel est actionné un moyen déclenchant une intervention automatique telle que la coupure d'alimentation de l'émetteur soumis à surveillance.

2. Procédé selon la revendication 1, caractérisé en ce que l'on contrôle les émissions de telle sorte que toute défaillance due à un vieillissement ou pour toute autre raison, peut être immédiatement détectée et signalée lorsqu'elle atteint un niveau prédéterminé.

3. Dispositif de détection, contrôle et surveillance d'un émetteur de radiations électromagnétiques, et du type comprenant deux capteurs (3, 4), une série de filtres (6, 10) sensibles aux radiations à contrôler, et des moyens de mesure et comparaison entre les courants débités par les capteurs, caractérisé en ce que ce dispositif est agencé pour la mise en oeuvre du procédé selon l'une des revendications précédentes.

4. Dispositif permettant de détecter, contrôler et surveiller en permanence un émetteur de radiations électromagnétiques de longueur d'onde se situant au voisinage de la lumière visible, notamment dans l'ultraviolet et utilisées plus spécialement à des fins germicides et d'assainissement d'ambiance et d'atmosphère, plus particulièrement en milieux sensibles tels que locaux médicaux, hospitaliers ou de traitement de produits alimentaires, installation de climatisation d'air ou piscines, et le dispositif est caractérisé en ce qu'il comporte deux cellules photoélectriques (3, 4) disposées dans le champ du rayonnement surveillé, une pluralité de filtres (6, 10) étant interposés dans ledit champ entre l'émetteur (2) et les cellules , et un premier filtre ou groupe de filtres (7, 8) étant aptes à atténuer ou arrêter les radiations marginales voisines de la longueur d'onde surveillée et se situant dans le spectre de la lumière visible, un second filtre (10) étant apte à transformer l'émission du rayonnement à surveiller en lumière visible apte à être détecté par la première cellule photoélectrique (3), tandis que la seconde cellule photoélectrique (4) comporte seule un filtre (9) occultant le rayonnement visible résultant de ladite transformation , le rayonnement visible résultant de cette transformation n'étant reçu que par la première cellule, les courants voltaïques débités par les deux cellules photoélectriques étant d'intensité différente en raison de cette différence, ceci pour le fonctionnement normal de l'émetteur, et les deux courants voltaïques étant recueillis aux bornes d'un comparateur (11) programmé pour basculer un relais (12) lorsque le différentiel entre les deux courants descend en dessous d'une valeur de consigne déterminée.

5. Dispositif de contrôle et de surveillance selon la revendication 4, caractérisé en ce qu'il comporte un relais (12) apte à couper l'alimentation de l'émetteur (2) et à actionner parallèlement un ensemble de signalisations.

6. Dispositif selon l'une des revendication 4 ou 5, caractérisé en ce qu'il est associé en permanence à un émetteur ultraviolet, constitué lui-même d'un tube d'émission (2) ultraviolette intégré dans un carter de protection.

7. Dispositif selon la revendication 6, caractérisé en ce qu'il est monté à demeure et à proximité immédiate de l'émetteur (2) de radiations à surveiller, tandis qu'au moins les cellules photoélectriques (3, 4) comportent des moyens propres à assurer leur déplacement, depuis une position effacée vers une position active dans laquelle elles sont situées dans le champ du rayonnement à surveiller.

8. Dispositif selon l'une des revendications 5 à 7, caractérisé en ce qu'il comporte un ensemble de composants monté sur un socle lui-même aimanté permettant de le positionner sur le carter métallique du boîtier d'un appareil à émission de radiations U.V..

9. Dispositif selon l'une des revendications 4 à 8, caractérisé en ce qu'il comporte un potentiomètre à axe fendu (26) pour le réglage du seuil de sensibilité, c'est-à-dire de l'intensité de radiations en dessous duquel l'intensité des radiations est considérée comme insuffisante et défectueuse.

10. Dispositif selon l'une des revendications 4 à 9, caractérisé en ce qu'il comporte un relais (12) apte à couper sa propre alimentation lorsque l'émission soumise à surveillance descend en dessous du seuil de consigne déterminé, l'appareil étant monté en série en amont de l'appareil émetteur, la coupure de l'alimentation entraînant par conséquent l'arrêt de l'appareil émetteur lui-même.

11. Dispositif selon l'une des revendications 4 à 10, caractérisé en ce qu'il comporte un ensemble porteur des composants électroniques, de la connectique, des moyens d'alarme et des moyens de protection rassemblés sur un socle comportant une semelle aimantée permettant le positionnement sur le carter métallique de l'appareil d'émission U.V., et le socle est associé à un boîtier séparé relié audit socle par une liaison filaire, le boîtier comportant les deux cellules et les filtres, aptes à être positionnés dans le champ des radiations U.V., ce boîtier comportant également une semelle aimantée pour sa fixation.

12. Dispositif selon l'une des revendications 4 à 11, caractérisé en ce qu'il est relié par un circuit de signalisation à une console centrale de commande et de surveillance regroupant les signaux provenant de l'ensemble des appareil U.V. appartenant à une même unité.

13. Dispositif pour la télécommande d'un pluralité de tubes émetteurs de radiations U.V., disposés en batterie, caractérisé en ce que chaque tube émetteur appartenant à ladite batterie (30) est relié à un poste central de surveillance (34) par un guide d'onde (31, 31'), notamment en fibre optique, chaque guide étant spécifique à un tube et comportant une extrémité distale (32) située face audit tube et une extrémité proximale (33) apte à délivrer la radiation transmise au niveau du poste central (34), ce dernier étant ainsi apte à recevoir les radiations émises et transmises par lesdits guides d'onde (31) par chacun des tubes, et le poste central est pourvu d'un ensemble de transducteurs photovoltaïques, notamment cellules photoélectriques, pourvus de filtres qui fonctionnent conformément à l'une des revendications 1 ou 2 ci-dessus, le poste central comportant des moyens mécanique, notamment un moteur électrique propre à positionner successivement ledit ensemble (35) de transducteurs photvoltaïques face à l'extrémité proximale de chacun des guides d'onde en permettant à chaque fois l'alimentation desdits transducteurs en radiation provenant successivement de chacun des tubes.

14. Dispositif selon la revendication 13, caractérisé en ce que les extrémités proximales des guides d'onde sont disposées selon une ligne parallèle au parcours de l'entrée de l'ensemble (35) des transducteurs photovoltaïques, et notamment lesdites extrémités proximales des guides d'onde sont disposées en cercle, l'ensemble (35) de transducteurs photovoltaïques étant disposé en position radiale et étant animé d'un mouvement angulaire pas-à-pas lui permettant de venir se positionner successivement en position d'interrogation devant chacune desdites extrémités proximales des guides d'onde.

15. Dispositif pour la télécommande d'une pluralité de tubes émetteurs de radiations U.V., disposés en batterie, caractérisé en ce que chaque tube comporte un ensemble de transducteurs photovoltaïques jumelés, notamment des cellules photoélectriques, pourvus de filtres qui fonctionnent conformément à l'une des revendications 1 ou 2, et chaque ensemble est relié à un poste central par un circuit approprié spécifique aux tubes correspondants, le poste central étant apte à comparer les caractéristiques des tensions recueillies à la sortie desdits ensembles transducteurs photovoltaïques pour en déduire l'état de fonctionnement des tubes correspondants.

16. Dispositif selon l'une des revendications 4 à 11, caractérisé en ce que l'ensemble des filtres et cellules (3, 4, 6, 10) est disposé dans le champ rayonné par l'émetteur ultraviolet, à l'intérieur de l'appareil mais non en contact direct avec le tube U.V. de façon à éviter la destruction rapide des cellules (3, 4).

## Patentansprüche

1. Verfahren zum Überwachen von magnetische Strahlung emittierenden Gegenständen, insbesondere von Strahlung sehr kurzer Wellenlänge im Bereich des sichtbaren Lichts, zum Beispiel einer Strahlung im Ultraviolettbereich,
dadurch gekennzeichnet, daß man einen ersten (3) und einen zweiten (4) Sensor, bestehend jeweils aus einem Meßwandler, insbesondere des photovoltaischen Typs, der zu überwachenden Strahlung aussetzt und vor den Sensoren eine Reihe von Filtern (6, 10) anordnet, von denen ein erstes Filter oder eine erste Filtergruppe (7, 8) in der Lage ist, Randstrahlung in der Nähe der zu überwachenden Wellenlänge im Bereich des Spektrums des sichtbaren Lichts zu dämpfen oder abzufangen, und ein zweites Filter (10) in der Lage ist, die Emission der zu überwachenden Strahlung in sichtbares Licht umzuwandeln, das von der ersten photoelektrischen Zelle (3) erfaßt werden kann, während die zweite photoelektrische Zelle (4) nur ein Filter (9) aufweist, das die durch die vorgenannte Umwandlung erhaltene sichtbare Strahlung unterbricht, so daß die aus der genannten Umwandlung resultierende sichtbare Strahlung nur von der ersten Zelle empfangen wird, daß man die von den beiden Meßwandlern jeweils ausgegebenen Ströme vergleicht, wobei die Differenz zwischen den beiden Strömen proportional zur Intensität der überwachten Strahlung ist, was die Anzeige oder Überwachung dieser Intensität ermöglicht, und daß man einen unteren Schwellwert für diese Differenz festlegt, unterhalb dessen Mittel betätigt werden, die einen automatischen Eingriff auslösen, zum Beispiel die Stromversorgung des überwachten, emittierenden Gegenstandes unterbrechen.

2. Verfahren nach Anspruch 1, dadurch gekennzeichnet, daß man die emittierte Strahlung derart überwacht, daß jeder Ausfall sofort erkannt und bei Erreichen eines vorbestimmten Wertes angezeigt werden kann.

3. Vorrichtung zum Erfassen und Überwachen eines elektromagnetische Strahlung emittierenden Gegenstandes mit zwei Sensoren (3, 4), einer Reihe von für die zu überwachende Strahlung empfindlichen Filtern (6, 10) und Mitteln zum Messen und Vergleichen der Ausgangsströme der Sensoren, dadurch gekennzeichnet, daß die Vorrichtung zur Durchführung des Verfahrens gemäß einem der vorhergehenden Ansprüche geeignet ist.

4. Vorrichtung zum Erfassen und ständigen Überwachen eines Gegenstandes, der elektromagnetische Strahlung einer Wellenlänge in der Nähe des sichtbaren Lichts, insbesondere im Ultraviolettbereich, emittiert, insbesondere für Zwecke der Keimabtötung und der Reinhaltung der Umgebung und der Luft, besonders in kritischen Bereichen wie medizinischen Räumen, Krankenhausbereichen oder bei der Behandlung von Lebensmitteln, in Klima- und Schwimmbadanlagen, dadurch gekennzeichnet, daß sie zwei im Strahlungsfeld der überwachten Strahlung angeordnete photoelektrische Zellen (3, 4) umfaßt, wobei eine Vielzahl von Filtern (6, 10) innerhalb des Strahlungsfeldes zwischen dem emittierenden Gegenstand (2) und den Zellen angeordnet ist und ein erstes Filter oder eine erste Filtergruppe (7, 8) in der Lage ist, Randstrahlung in der Nähe der zu überwachenden Wellenlänge im Bereich des Spektrums des sichtbaren Lichts zu dämpfen oder abzufangen, und ein zweites Filter (10) in der Lage ist, die Emission der zu überwachenden Strahlung in sichtbares Licht umzuwandeln, das von der ersten photoelektrischen Zelle (3) erfaßt werden kann, während die zweite photoelektrische Zelle (4) nur ein Filter (9) aufweist, das die durch die vorgenannte Umwandlung erhaltene sichtbare Strahlung unterbricht, so daß die aus der genannten Umwandlung resultierende sichtbare Strahlung nur von der ersten Zelle empfangen wird, wobei die von den beiden photoelektrischen Zellen ausgegebenen voltaischen Ströme wegen dieser Differenz bei normalem Betrieb des emittierenden Gegenstandes eine unterschiedliche Intensität aufweisen und an den Klemmen eines Vergleichers (11) anliegen, der derart programmiert ist, daß ein Relais (12) schaltet, wenn die Differenz zwischen den beiden Strömen unter einen vorbestimmten Referenzwert absinkt.

5. Überwachungsvorrichtung nach Anspruch 4, dadurch gekennzeichnet, daß sie ein Relais (12) umfaßt, das in der Lage ist, die Stromversorgung des emittierenden Gegenstandes (2) zu unterbrechen und parallel dazu eine Anzeigeeinrichtung zu aktivieren.

6. Vorrichtung nach einem der Ansprüche 4 oder 5, dadurch gekennzeichnet, daß sie ständig einem ultraviolette Strahlung emittierenden Gegenstand zugeordnet ist, wobei letzterer aus einer in einem Schutzgehäuse integrierten Ultraviolettlampe (2) besteht.

7. Vorrichtung nach Anspruch 6, dadurch gekennzeichnet, daß sie in unmittelbarer Nähe des die zu überwachenden Strahlung emittierenden Gegenstandes (2) fest angebracht ist, während mindestens die photoelektrischen Zellen (3, 4) Mittel umfassen, die ihre Bewegung von einer unwirksamen Stellung in eine aktive Stellung innerhalb des Strahlungsfeldes der zu überwachenden Strahlung ermöglichen.

8. Vorrichtung nach einem der Ansprüche 5 bis 7, dadurch gekennzeichnet, daß ihre Bestandteile auf einem seinerseits magnetischen Sockel montiert sind, mittels dessen die Vorrichtung am metallischen Rahmen des Gehäuses eines UV-Strahlung emittierenden Geräts angebracht werden kann.

9. Vorrichtung nach einem der Ansprüche 4 bis 8, dadurch gekennzeichnet, daß sie ein Potentiometer mit geteilter Achse (26) zur Regelung der Empfindlichkeitsschwelle, d.h. der Strahlungsintensität umfaßt, unterhalb derer die Strahlungsintensität als unzureichend und fehlerhaft angesehen wird.

10. Vorrichtung nach einem der Ansprüche 4 bis 9, dadurch gekennzeichnet, daß sie ein Relais (12) umfaßt, das in der Lage ist, seine eigene Stromzufuhr zu unterbrechen, wenn die überwachte Strahlung unter den vorbestimmten Referenzschwellwert absinkt, wobei die Vorrichtung dem die Strahlung emittierenden Gerät in Reihe vorgeschaltet ist, so daß bei Unterbrechung der Stromzufuhr infolgedessen auch das die Strahlung emittierende Gerät selbst abgeschaltet wird.

11. Vorrichtung nach einem der Ansprüche 4 bis 10, dadurch gekennzeichnet, daß sie eine Einheit zur Aufnahme der elektronischen Komponenten, der Anschlüsse, der Alarmeinrichtungen und der Schutzeinrichtungen umfaßt, die zusammen auf einem Sockel mit magnetischer Fußplatte angeordnet sind, mittels derer die Einheit am metallischen Gehäuse des die UV-Strahlung emittierenden Geräts angebracht werden kann, und daß der Sockel einem getrennten, über eine Leiterverbindung mit dem Sockel verbundenen Gehäuse zugeordnet ist, das die beiden Zellen und die Filter enthält, die in dem UV-Strahlungsfeld positioniert werden können, und der Sockel für seine Befestigung ebenfalls eine magnetische Fußplatte aufweist.

12. Vorrichtung nach einem der Ansprüche 4 bis 11, dadurch gekennzeichnet, daß sie über eine Signalschaltung mit einer zentralen Steuer- und Überwachungstafel verbunden ist, auf der alle von den zu einer Einheit gehörenden UV-Geräten kommenden Signale zusammengefaßt sind.

13. Vorrichtung zur Fernsteuerung einer Vielzahl von UV-Strahlungslampen einer Lampengruppe, dadurch gekennzeichnet, daß jede zu der Gruppe (30) gehörende Strahlungslampe über einen Wellenleiter (31, 31'), insbesondere eine Lichtleitfaser, mit einer zentralen Überwachungsstation (34) verbunden ist, wobei jeder der Wellenleiter einer Lampe speziell zugeordnet ist und ein der Lampe gegenüberliegendes äußeres Ende (32) sowie ein inneres Ende (33) aufweist, das die übermittelte Strahlung im Bereich der zentralen Überwachungsstation (34) abgeben kann, wobei letztere die emittierte und von den Wellenleitern (31) der einzelnen Lampen übermittelte Strahlung empfangen kann und eine Gruppe photovoltaischer Meßwandler, insbesondere photoelektrischer Zellen, aufweist, die mit entsprechend einem der vorstehenden Ansprüche 1 oder 2 arbeitenden Filtern ausgestattet sind, wobei die zentrale Überwachungsstation mechanische Mittel, insbesondere einen Elektromotor, aufweist, mittels dessen die Gruppe (35) der photovoltaischen Meßwandler nacheinander in eine Position gegenüber dem inneren Ende der einzelnen Meßwandler gebracht werden kann und die Meßwandler gleichzeitig jedesmal mit nacheinander von den einzelnen Lampen kommender Strahlung beaufschlagt werden können.

14. Vorrichtung nach Anspruch 14, dadurch gekennzeichnet, daß die inneren Enden der Wellenleiter entlang einer parallel zur Bewegungslinie des Eingangs der Gruppe (35) der photovoltaischen Meßwandler verlaufenden Linie angeordnet sind und daß insbesondere die inneren Enden der Wellenleiter in einem Kreis angeordnet sind, wobei die Gruppe (35) der photovoltaischen Meßwandler radial angeordnet ist und schrittweise eine Schwenkbewegung vollführt, mittels derer sie nacheinander in eine Abfrageposition vor jedes der inneren Enden der Wellenleiter gebracht werden kann.

15. Vorrichtung zur Fernsteuerung einer Vielzahl von UV-Strahlungslampen einer Lampengruppe, dadurch gekennzeichnet, daß jede Lampe eine Gruppe fest miteinander verbundener photovoltaischer Meßwandler, insbesondere photoelektrischer Zellen umfaßt, die mit entsprechend einem der Ansprüche 1 oder 2 arbeitenden Filtern ausgestattet sind, und daß jede Gruppe über eine für die betreffenden Lampen spezielle, geeignete Schaltung mit einer zentralen Überwachungsstation verbunden ist, die in der Lage ist, die am Ausgang der photovoltaischen Wandlergruppen jeweils anliegenden Spannungsmerkmale zu vergleichen, um daraus den Funktionszustand der entsprechenden Lampen abzuleiten.

16. Vorrichtung nach einem der Ansprüche 4 bis 11, dadurch gekennzeichnet, daß die Gruppe der Filter und Zellen (3, 4, 6, 10) innerhalb des Geräts im Strahlungsfeld der Ultraviolettlampe, aber ohne direkten Kontakt zur UV-Lampe angeordnet ist, um eine schnelle Zerstörung der Zellen (3, 4) zu vermeiden.

## Claims

1. Method for controlling the flowrate and intensity of an electromagnetic emission, particularly of very short wavelength, in the vicinity of visible light, such as an emission of waves in the ultraviolet,
characterized in that there are exposed to the radiations to be controlled a first (3) and a second (4) sensor each constituted by a transducer particularly of the photovoltaic type, while there is interposed in front of said sensors a series of filters (6, 10), a first filter or group of filters (7, 8) being adapted to attenuate or stop the marginal radiations close to the wave-length supervised and located in the visible light spectrum, a second filter (10) being adapted to transform the emission of the radiation to be supervised into visible light adapted to be detected by the first photoelectric cell (3), while the second photoelectric cell (4) alone comprises a filter (9) occulting the visible radiation resulting from said transformation, the visible radiation resulting from this transformation being received only by the first cell, the currents delivered by each of these transducers are compared, the differential between the two currents being proportional to the intensity of the radiation supervised, which makes it possible to control or display this intensity, a minimum reference threshold of said differential is determined, below which is actuated a means triggering off an automatic intervention such as the cut-off of supply of the emitter subjected to supervision.

2. Method according to Claim 1, characterized in that the emissions are controlled so that any failure due to ageing or for any other reason may be immediately detected and signalled when it attains a predetermined level.

3. Device for detecting, controlling and supervising an electromagnetic radiation emitter, and of the type comprising two sensors (3, 4), a series of filters (6, 10) sensitive to the radiations to be controlled, and means for measuring and comparison between the currents delivered by the sensors, characterized in that this device is arranged to carry out the method according to one of the preceding Claims.

4. Device for permanently detecting, controlling and supervising an electromagnetic radiation emitter of wave-length located in the vicinity of visible light, particularly in the ultraviolet and used more especially for germicidal and environment and atmosphere purification purposes, more particularly in sensitive environments such as medical, hospital or food-processing premises, air-conditioning installations or swimming pools, and the device is characterized in that it comprises two photoelectric cells (3, 4) disposed in the field of the radiation supervised, a plurality of filters (6, 10) being interposed in said field between the emitter (2) and the cells, and a first filter or group of filters (7, 8) being adapted to attenuate or stop the marginal radiations close to the wave-length supervised and located in the visible light spectrum, a second filter (10) being adapted to transform the emission of the radiation to be supervised into visible light adapted to be detected by the first photoelectric cell (3), while the second photoelectric cell (4) alone comprises a filter (9) occulting the visible radiation resulting from said transformation, the visible radiation resulting from this transformation beinq received only by the first cell, the voltaic currents delivered by the two photoelectric cells being of different intensity due to this difference, this for normal functioning of the emitter, and the two voltaic currents being collected at the terminals of a comparator (11) programmed to trip a relay (12) when the differential between the two currents descends below a determined reference value.

5. Control and supervision device according to Claim 4, characterized in that it comprises a relay (12) adapted to cut off supply of the emitter (2) and, in parallel, to actuate a signalization assembly.

6. Device according to one of Claims 4 or 5, characterized in that it is permanently associated with an ultraviolet emitter, itself constituted by an ultraviolet emission tube (2) integrated in a protective housing.

7. Device according to Claim 6, characterized in that it is permanently mounted in the immediate proximity of the emitter (2) of radiations to be supervised, while at least the photoelectric cells (3, 4) comprise means adapted to ensure displacement thereof from a retracted position towards an active position in which they are located in the field of the radiation to be supervised.

8. Device according to one of Claims 5 to 7, characterized in that it comprises an assembly of components mounted on a base itself magnetized, enabling it to be positioned on the metal housing of the casing of an U.V. radiation emission apparatus.

9. Device according to one of Claims 4 to 8, characterized in that it comprises a split pin potentiometer (26) for adjusting the threshold of sensitivity, i.e. the intensity of radiations below which the intensity of the radiations is considered as insufficient and defective.

10. Device according to one of Claims 4 to 9, characterized in that it comprises a relay (12) adapted to cut off its own supply when the emission subjected to supervision descends below the determined reference threshold, the apparatus being mounted in series upstream of the emitter apparatus, the fact of the supply being cut off consequently causing stop of the emitter apparatus itself.

11. Device according to one of Claims 4 to 10, characterized in that it comprises an assembly bearing electronic components, connections, alarm means and protection means gathered together on a base comprising a magnetized sole allowing positioning on the metal casing of the U.V. emission apparatus, and the base is associated with a separate casing connected to said base by a wire connection, the casing comprising the two cells and the filters, adapted to be positioned in the field of the U.V. radiations, this casing also comprising a magnetized sole for fixation thereof.

12. Device according to one of Claims 4 to 11, characterized in that it is connected by a signalling circuit to a central control and supervision console grouping the signals coming from the assembly of the U.V. apparatus belonging to the same unit.

13. Device for the remote control of a plurality of U.V. radiation emitter tubes disposed in battery, characterized in that each emitter tube belonging to said battery (30) is connected to a central supervision station (34) by a wave guide (31, 31'), particularly in optical fiber form, each guide being specific to a tube and comprising a distal end (32) located opposite said tube and a proximal end (33) adapted to deliver the radiation transmitted at the level of the central station (34), this latter thus being adapted to receive the radiations emitted and transmitted by said wave guides (31) by each of the tubes, and the central station is provided with an assembly of photovoltaic transducers, particularly photoelectric cells, provided with filters which function in accordance with one of Claims 1 or 2 hereinabove, the central station comprising mechanical means; particularly an electric motor adapted to position said assembly (35) of photovoltaic transducers successively opposite the proximal end of each of the wave guides allowing, each time, the supply of said transducers with radiation coming successively from each of the tubes.

14. Device according to Claim 13, characterized in that the proximal ends of the wave guides are disposed in a line parallel to the path of the input of the assembly (35) of the photovoltaic transducers, and in particular said proximal ends of the wave guides are disposed in a circle, the assembly (35) of photovoltaic transducers being disposed in radial position and being animated by an angular step-by-step movement enabling it to come successively into position of interrogation in front of each of said proximal ends of the wave guides.

15. Device for the remote-control of a plurality of U.V. radiation emitter tubes, disposed in battery, characterized in that each tube comprises an assembly or twinned photovoltaic transducers, particularly photoelectric cells, provided with filters which function in accordance with one of Claims 1 or 2, and each assembly is connected to a central station by an appropriate circuit specific to the corresponding tubes, the central station being adapted to compare the characteristics of the voltages collected at the output of said photovoltaic transducer assemblies in order to deduce the state of functioning of the corresponding tubes.

16. Device according to one of Claims 4 to 11, characterized in that the assembly of the filters and cells (3, 4, 6, 10) is disposed in the field radiated by the ultraviolet emitter, inside the apparatus but not in direct contact with the U.V. tube so as to avoid rapid destruction of the cells (3, 4).
